# EUROPEAN PATENT APPLICATION

(11) **EP 2 606 855 A1**
(43) Date of publication of application: **26.06.2013**
(21) Application number: 11195647.0
(22) Date of filing: 23.12.2011
(51) Int. Cl.: A61F 2/24

(54) **Prosthetic heart valve**

(71) Applicant: Ludwig-Maximilians-Universität München, 80539 München (DE)
(72) Inventor: Bombien, René, 81373 München (DE); Akra, Bassil, 82140 Olching (DE); Überfuhr, Peter, 81241 München (DE)
(74) Representative: Lucke, Andreas

(57) **Abstract**

Disclosed herein is a prosthetic heart valve comprising a frame member 12, and at least two flaps 14a-c attached to said frame member 12 and allowing blood flow in a first direction but restricting blood flow in a second direction opposite to said first direction. The frame member 12 is adapted to acquire a first configuration and a second configuration. The restriction of blood flow when the frame member is in the first configuration is less than when the frame member 12 is in the second configuration. The prosthetic heart valve 10 further comprises means allowing the frame member 12 to gradually or stepwisely change between the first and second configurations after implantation.

## Description

### Field of the Invention

The present invention is in the field of medical technology. In particular, the present invention relates to a prosthetic heart valve that can be implanted in a patient.

### Related Prior Art

A prosthetic heart valve is a device implanted in the heart of a patient if one of the four heart valves malfunctions. Generally, there are two classes of prosthetic heart valves, namely mechanical and biological heart valves, where the latter are sometimes also referred to as "tissue heart valves".

Mechanical heart valves are completely artificial, man-made devices that have been devised and implanted since the 1950's. The major advantage of mechanical heart valves is their lifetime, which exceeds the maximum lifetime of a patient by many times. However, all current mechanical heart valves require lifelong treatment with anti-coagulation agents, such as vitamin-K antagonists.

Biological prosthetic heart valves, on the other hand, do not require the use of anti-coagulant agents, since the improved blood flow dynamics results in less coagulation. However, unfortunately the lifetime of biological prosthetic heart valves is limited.

From US 4,364,127, a prosthetic heart valve constructed of hemo-compatible materials is known. The prosthetic heart valve is anatomically and functionally similar to a natural aortic valve. It is of a tri-leaflet type, where the leaflets are heat-set in a partially open position to thereby reduce pressure required to open the leaflets in response to blood flowing therethrough.

Irrespectively of the specific design of the prosthetic heart valve, not only the implantation itself but also the recovery period from the implantation is a critical phase putting considerable strain to the patient. After implantation, the risk of a patient's heart failure is increased, and usually, an extended intensive care unit stay with circulatory support, such as intra-aortic balloon pump support and inotropic medication is necessary.

### Summary of the Invention

The problem underlying the invention is to provide a prosthetic heart valve that helps to alleviate the strain to the patient during recovery from the implantation.

This problem is solved by a prosthetic heart valve according to claim 1. Preferable embodiments are defined in the dependent claims.

According to the invention, the prosthetic heart valve comprises a frame member and at least two flaps attached to said frame member and allowing blood flow in a first direction but restricting blood flow in a second direction opposite to said first direction, to thereby provide a valve function. According to the invention, the frame member is adapted to acquire a first configuration and a second configuration, wherein the restriction of blood flow when the frame member is in the first configuration is less than when the frame member is in the second configuration. Further, the prosthetic heart valve comprises means allowing the frame member to gradually or stepwisely change between said first and second configurations after implant.

Accordingly, the prosthetic heart valve can be implanted while the frame member is in a first configuration, which is a configuration in which the restriction of blood flow in the second direction is decreased as compared to the second configuration. In other words, the first configuration of the frame member causes the prosthetic heart valve to acquire an "artificial insufficiency". The purpose of this "artificial insufficiency" is that the behavior of the artificial heart valve immediately after implantation is more similar to that of the diseased or malfunctional heart valve that has been replaced, such as to alleviate the rather dramatic change in the hemodynamics that would normally occur if an insufficient heart valve is replaced by a prosthetic heart valve that immediately restores the efficiency of a healthy heart valve. In particular, the frame member in its first configuration may prevent a physiological closure of the valve. This does not only lead to a decrease in the blood flow restriction in the second direction but may also decrease the flow resistance of blood flow in the first direction, thus decreasing the pumping work that needs to be done by the heart. The invention is motivated by the understanding that the rather dramatic change of the hemodynamic behavior after implantation causes a significant part of the complications during the recovery time.

Further, the prosthetic heart valve comprises means allowing the frame member to change between said first and second configurations, thereby continuously increasing the capability of the prosthetic heart valve to restrict the blood flow in the second direction, or, in other words, the efficiency of the heart valve. Accordingly, the patient can accustom to the gradually or stepwisely increasing heart valve efficiency. For example, the artificial insufficiency can be improved to normal efficiency in a period of say 1 to 12 weeks, thereby alleviating the recovery phase for the patient.

The inventors have found that while there are in principle many conceivable ways to manipulate the efficiency of a prosthetic heart valve, manipulating a frame member to which the flaps are attached according to the present understanding is the simplest and most robust way to achieve this, and in particular better than manipulating the properties of the flaps themselves. However, based on this understanding, there are many different ways how the frame member can be chosen and how the configurational variability can be achieved.

In a preferred embodiment, the frame member has a circumference along which said at least two flaps are arranged, and the length of the circumference in the first configuration is larger than in the second configuration. By increasing the circumference of the frame member in the first configuration, a complete closure of the valve can be prevented or, in other words, a gap for blood flow in the second direction can be formed, thereby reducing the restriction of blood flow in the second direction.

In a particularly advantageous embodiment, the frame member is ring-shaped and in particular in the shape of a closed or open ring, and the blood flows through said closed or open ring when the prosthetic heart valve is implanted. Herein, the "open ring" can be thought of as a ring having a gap that can be narrowed or extended to thereby decrease or increase the diameter of the ring, respectively. However, the ring may also have a closed shape, where a variation of the circumference can be achieved for example by one end of the ring plunging into a cavity in the second end in a telescopic manner. Note that the ring need not necessarily be circular but could also have an oval shape.

In one embodiment, the means for allowing the frame member to change between said first and second configurations comprises mechanical means, in particular threaded means, like a screw, a worm gear or the like. Preferably, the mechanical means are configured to be actuated endoscopically. This way, the efficiency of the prosthetic heart valve can be increased as appropriate by a minimal invasion.

Alternatively, the means for allowing the frame member to change between the first and second configurations may comprise electromechanical means, such as a micro-actuator coupled to the frame member. The micro-actuator can for example be programmed to carry out a predetermined change of configuration in a predetermined time, such as to increase the efficiency according to a suitable time curve defined by a cardiac surgeon or cardiac specialist. In the alternative, the micro-actuator can be controlled remotely by means of an RF-link or a cable-link.

Further, the means for allowing the frame member to change between the first and second configurations can also employ electromagnetic means that can be actuated by applying electrical and/or magnetic fields from outside a patient in which the prosthetic heart valve is implanted. In these embodiments, the means may involve components that are mechanical in nature, but are actuated from outside the patient by means of electrical or magnetic fields, thereby avoiding any invasion whatsoever. Finally, it is possible to employ frame members comprising a shape memory material that can make a transition between two different geometric configurations. While traditionally, shape memory materials have been used that change their configuration in a reversible way in response to temperature, some shape memory materials may change their configuration in response to application of a current.

In a preferred embodiment, a gap of variable size is formed in the frame member, wherein the gap has a larger size when said frame member acquires the first configuration and has a smaller or zero size when the frame member acquires the second configuration. Preferably, the gap is located adjacent to a commissure of two adjacent flaps. Accordingly, closing the gap may lead to a complete or nearly complete closure of the valve for flow in the second direction by means of the flaps, while the gap in the frame member may lead to a gap between adjacent flaps that causes a reduction in the efficiency of the valve.

In a preferred embodiment, a spacer element is provided in the gap, wherein the spacer element has a variable size and the frame is biased into a closed position of the gap. In this embodiment, the variable size spacer element forms part of the means for allowing the frame member to change between the first and second configurations. Namely, as the size of the spacer element decreases, due to the biasing force the gap automatically closes, thereby causing a transition from the first to the second configuration.

Herein, the size of the spacer element can be actively varied, and in particular decreased, in a number of different ways. For example, the size of the spacer element can be varied by a mechanical control, and in particular, by endoscopic actuation of a threaded element, such as a screw. Alternatively, the size of the spacer element can be varied by an electromagnetic control, and in particular, by an electromagnetic control from outside the patient in which the heart valve is implanted. Finally, a micro-actuator may be included in or coupled with the spacer element to vary the size of the spacer element and hence to change the configuration of the frame member.

However, the size of the spacer element may also be passively decreased after implantation, to thereby cause a transition from the first to the second configuration. For example, the spacer element may be made of a biodegradable spacer material. After implantation, the spacer element will gradually disintegrate by natural processes, thereby leading to a decrease in size and hence to a gradual closure of the gap. This embodiment is particularly attractive due to its simplicity, low cost and robustness.

The prosthetic heart valve may preferably have two or three flaps, and the flaps may be cuspidal or semi-lunar flaps. In particular, the flap structure may be similar to or identical with that of a tri-cuspidal or a mitral heart valve.

In a preferred embodiment, the flaps are made of biological tissue that is attached to the frame member. The tissue can for example be grown by tissue engineering. However, the concept of the invention is also applicable to mechanical heart valves.

### Short Description of the Figures

- Fig. 1: is a perspective view of a prosthetic heart valve according to one embodiment of the invention including biodegradable spacers.

- Fig. 2: is a further perspective view of the prosthetic heart valve of Fig. 1.
- Fig. 3: is a perspective sectional view of a prosthetic heart valve according a further embodiment of the invention.
- Fig. 4: is a perspective view of a prosthetic heart valve according a further embodiment of the invention allowing for a change between the first and second configurations due to manipulation by means of a mechanical tool.
- Fig. 5: is a perspective view of a prosthetic heart valve according a further embodiment of the invention comprising a frame member made of a shape memory material.

### Description of the Preferred Embodiment

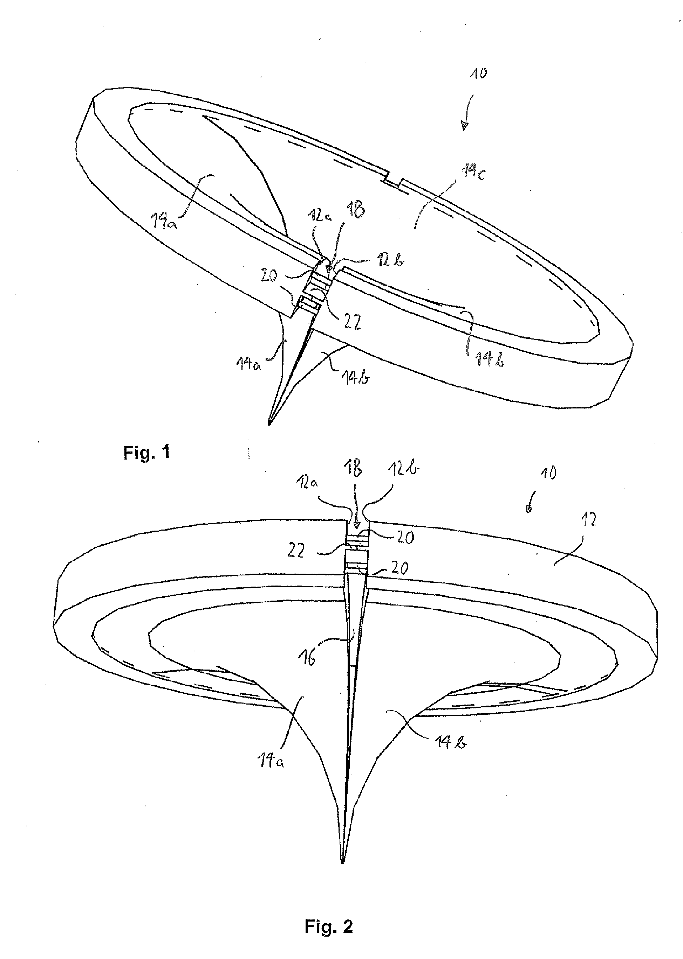

In Figs. 1 and 2, two perspective views of a prosthetic heart valve 10 according to a first embodiment of the invention are shown. As is seen in Figs. 1 and 2, the prosthetic heart valve 10 comprises a frame member 12, to which three flaps 14a-c are attached. The flaps 14a-c are cuspidal flaps, giving rise to a shape that is similar to a natural tri-cuspidal heart valve. The flaps 14a-c may be made of biological and/or non-biological material. In case of biological material, the flaps 14a-c could be homografts or xenografts. In a particularly preferred embodiment, the flaps 14a-c, however, could be made by tissue engineering. As is further seen in Figs. 1 and 2, the frame member 12 in this embodiment is ring-shaped, where the blood flows through the ring-shaped frame member 12 when the prosthetic heart valve 10 is implanted.

Within the ring-shaped frame member 12, a gap 18 is formed. Figs. 1 and 2 show the prosthetic heart valve 10 in a first configuration in which the gap 18 is kept open by two biodegradable spacers 20 that are arranged in the gap 18. The biodegradable spacers 20 counteract a biasing force applied by the ring-shaped frame member 12 that biases the frame member into a second configuration (not shown) in which the gap 18 is closed. A guiding means 22 is attached to one free end 12a of the ring-shaped frame member 12 and dives into a corresponding recess in the opposing free end 12b of the ring-shaped frame member 12 in a telescopic manner. Accordingly, when the frame member 12 moves from the first configuration shown in Figs. 1 and 2 to the second configuration by closing the gap 18, the guiding means 22 guides the free ends 12a,b of the ring-shaped frame member 12 upon approaching each other. Next, the function of the prosthetic heart valve 10 of Figs. 1 and 2 is explained. As mentioned before, Fig. 1 shows the first configuration of the ring-shaped frame member 12 in which the gap 18 is kept open by the biodegradable spacer elements 20. The heart valve 10 is implanted with the frame member 12 being in this first configuration. Due to the open gap 18, the flaps 14a-c of the heart valve do not completely close but form a predetermined gap 16 between two adjacent flaps 14a, 14b thereby causing an artificial and purposeful insufficiency of the heart valve 10. With particular reference to Fig. 2, it can be seen how the gap 18 in the ring-shaped frame 12 leads to a gap 16 at the commissure of the flaps 14a and 14b, hence leading to an insufficiency of the heart valve 10. This insufficiency of the heart valve 10 is similar to that of a diseased or malfunctional heart valve that is to be replaced by the prosthetic heart valve 10.

After implantation, the biodegradable spacer elements 20 disintegrate by a preferably natural process, thereby diminishing in size and gradually closing the gap 18 due to the biasing force of the frame member 12. Along with the gap 18 in the frame member 12, the gap 16 between the adjacent flaps 14a and 14b (see Fig. 2) decreases, so that the efficiency of the heart valve 10 is gradually increased. Note that Figs. 1 and 2 show the heart valve 10 with its flaps 14a-c in their closed state, i.e. in a state that should restrict blood flow in the "second direction" as mentioned above. However, even in this closed state of the flaps 14a-c, there is still a gap 16 at the commissure between the flaps 14a and 14b.

At the end of the disintegration of the spacer elements 20, which could, depending on the selected spacer elements 20, take between 1 and 12 weeks, the gap 18 is entirely closed. This corresponds to the second configuration of the frame member 12, in which the ends 12a,b of the open ring-shaped frame member 12 abut against each other. In the second configuration, the circumference of the ring-shaped frame member 12 has decreased as compared to the first configuration, and the prosthetic heart valve 10 acquires its maximum efficiency, since the gap 16 between the flaps 14a,b will be closed.

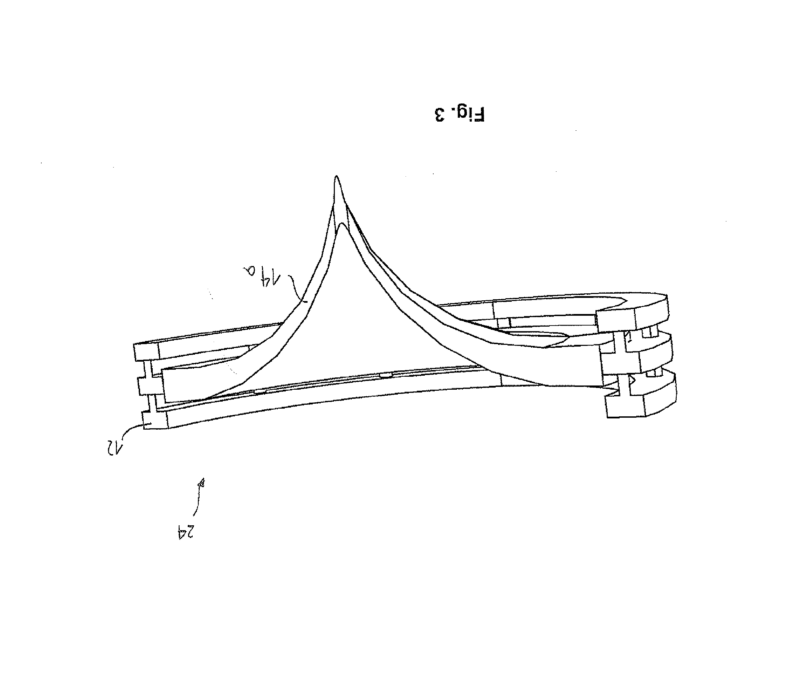

Fig. 3 shows a perspective sectional view of a similar heart valve 24. The heart valve 24 comprises a ring-shaped frame member 12 to which flaps 14 are attached.

The embodiment of Figs. 1 and 2 shows a very simple and elegant way of transforming the frame 12 from its first to its second configuration by providing spacer elements 20 in a gap 18 that may decrease in size. In the embodiment of Figs. 1 and 2, the size of the spacer elements 20 is decreased "passively" by biodegradation. This is particularly attractive, since it does not require any surgical invasion after the heart valve 10 is implanted in the patient. Nevertheless, it is also conceivable to provide for a spacer element having a size that can be actively varied, for example by a mechanical control, such as an endoscopic actuation of a threaded element, by electromagnetic control, in particular by an electromagnetic control from outside a patient in which the heart valve is implanted, or by a micro-actuator included in or coupled with a spacer element.

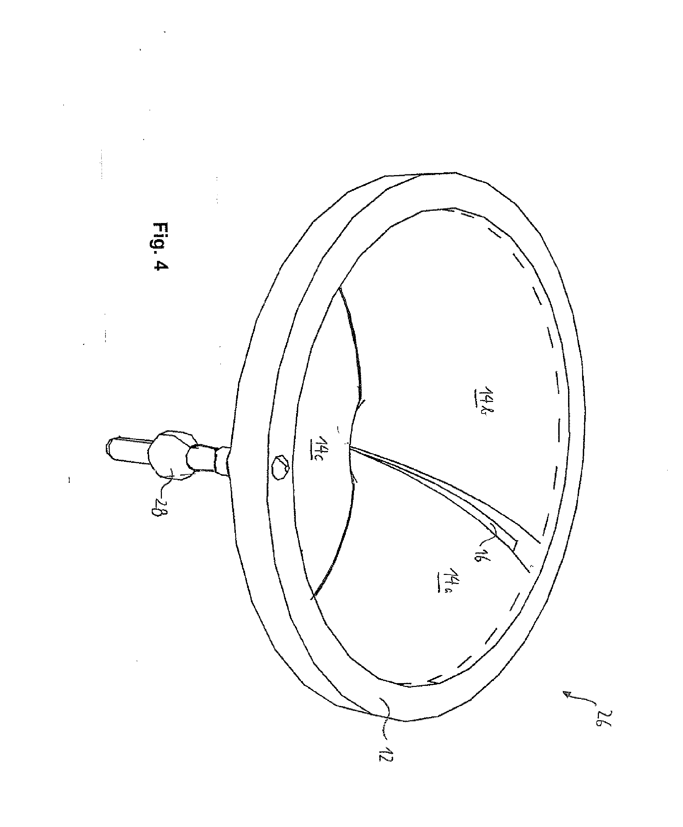

In an alternative embodiment, the frame member 12 may be changed between the first and second configurations using mechanical means that can be actuated endoscopically. Fig. 4 shows a further embodiment of a prosthetic heart valve 26 which is very similar to the prosthetic heart valve 14 of Figs. 1 and 2. Consequently, like components are designated with the same reference sign. The main difference between the prosthetic heart valve 26 of Fig. 4 and the prosthetic heart valve 10 of Figs. 1 and 2 is how the circumference of the frame member 12 is changed after implantation. According to Fig. 4, the circumference of the frame element 12 can be changed by actuation of a tool 28 that operates a screw mechanism (not shown) included in the frame member 12. The tool 28 can be operated in a minimally invasive way using an endoscope (not shown).

The prosthetic heart valve 26 of Fig. 4 thus allows adjusting the "artificial insufficiency" of the heart valve 26 in a controlled manner, for example based on the recovery process of the patient. This may in some cases be advantageous over the purely passive configuration change of Figs. 1 and 2, although at the price of a moderate endoscopical intervention. Hence, both embodiments have their merits.

Instead of employing mechanical means for allowing the frame member 12 to change between the first and second configurations, electromechanical means (not shown) may be employed. In particular, a micro-actuator coupled to the frame member 12 may be used, wherein the micro-actuator can be controlled remotely by means of an RF link or a cable link.

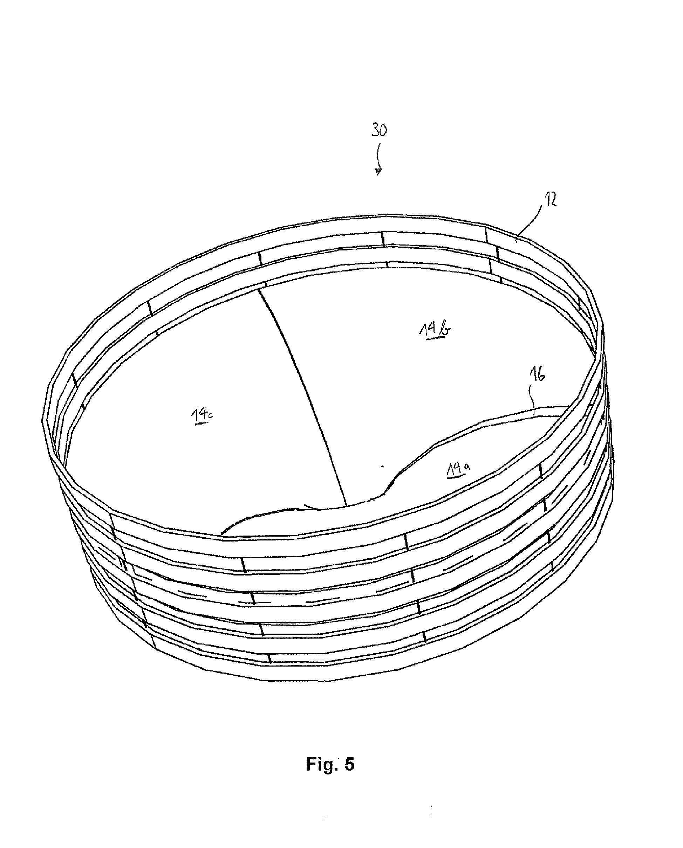

A further embodiment of a prosthetic heart valve 30 is shown in Fig. 5. In the embodiment of Fig. 5, again the circumference of a ring-shaped frame member 12 is varied between a first configuration as shown in Fig. 5 and a second configuration (not shown), in which the circumference is decreased. However, in the prosthetic heart valve 30 of Fig. 5, there is no gap in the ring element that is opened or closed. Instead, the ring member 12 is made of a shape memory material that can make a transition between two different geometric configurations. In particular, the material employed may be a material that shrinks and expands in response to application of an electric current. Accordingly, by applying a suitable electric current to the frame member 12 of the prosthetic heart valve 30 of Fig. 5 after implantation, the transition from the first configuration shown in Fig. 5 to a second configuration of smaller diameter (not shown) can be induced. Again, the current can be applied in a minimally invasive manner by means of an endoscope.

Since the efficiency of the heart valve increases gradually, a dramatic change in the hemodynamics can be alleviated that would normally occur if an insufficient heart valve is replaced by a prosthetic heart valve that immediately restores the efficiency of a healthy heart valve. Accordingly, the patient can gradually accustom to the increased heart valve efficiency, which makes the recovery phase easier and helps to avoid complications.

### REFERENCE SIGNS:

- 10: prosthetic heart valve
- 12: frame member
- 14a-c: cuspidal flap
- 16: gap between adjacent flaps 19a, 14b
- 18: gap in frame member 12
- 20: spacer element
- 22: guiding means
- 24: prosthetic heart valve
- 26: prosthetic heart valve
- 28: mechanical tool
- 30: prosthetic heart valve

## Claims

1. A prosthetic heart valve (10, 24, 26, 30), comprising:
- a frame member (12), and
- at least two flaps (14a,b,c) attached to said frame member (12) and allowing blood flow in a first direction but restricting blood flow in a second direction opposite to said first direction,
said frame member (12) being adapted to acquire a first configuration and a second configuration,
wherein said restriction of blood flow when the frame member (12) is in the first configuration is less than when the frame member (12) is in the second configuration, said prosthetic heart valve (10, 24, 26, 30) further comprising means (20) allowing the frame member (12) to gradually or stepwisely change between the first and second configurations after implantation.

2. The prosthetic heart valve (10, 24, 26, 30) of claim 1, wherein said frame member (12) has a circumference along which said at least two flaps (14a,b,c) are arranged, and wherein the length of said circumference in the first configuration is larger than in the second configuration.

3. The prosthetic heart valve (10, 24, 26, 30) of claim 1 or 2, wherein said frame member (12) is ring-shaped, and in particular in the shape of a closed or open ring,
and wherein the blood flows through said closed or open ring when said prosthetic heart valve (10, 24, 26, 30) is implanted.

4. The prosthetic heart valve (10, 24, 26, 30) of one of the preceding claims, wherein said means for allowing the frame member (12) to change between said first and second configurations comprises one or more of the following:
- mechanical means, in particular threaded means, and preferably threaded means that can be actuated endoscopically,
- electromechanical means, in particular a micro-actuator coupled to said frame member (12), wherein said micro-actuator can be controlled remotely by means of an RF- or a cable-link, or
- electromagnetic means that can be actuated by applying electrical and/or magnetic fields from outside a patient in which the prosthetic heart valve (10, 24, 26, 30) is implanted.

5. The prosthetic heart valve (10, 24, 26) of one of the preceding claims, wherein a gap (18) of variable size is formed in said frame member (12), wherein the gap (18) has a larger size when said frame member (12) acquires the first configuration and has a smaller or zero size when said frame member (12) acquires the second configuration.

6. The prosthetic heart valve (10, 24, 26) of claim 5, wherein said gap (18) is located adjacent to a commissure (16) of two adjacent flaps (14a,b).

7. The prosthetic heart valve (10) of claim 5 or 6, wherein a spacer element (20) is provided in said gap (18), said spacer element (20) having a variable size and the frame member (12) being biased into a closed position of the gap (18) when the prosthetic heart valve (10) is implanted.

8. The prosthetic heart valve (10) of claim 7, wherein the size of the spacer element (20) can be actively varied, and in particular decreased, by one or more of the following means:
- a mechanical control, and in particular by endoscopic actuation of a threaded element, such as a screw,
- an electromagnetic control, in particular an electromagnetic control from outside a patient in which the heart valve is implanted, or
- a micro-actuator included in or coupled with said spacer element (20).

9. The prosthetic heart valve (10) of claim 7, wherein the size of said spacer element (20) can be passively varied, and in particular decreased, after implantation, in particular by degradation of a biodegradable spacer material.

10. The prosthetic heart valve (10) of one of the preceding claims, wherein said at least two flaps (14a,b,c) are cuspidal flaps or semi-lunar flaps.

11. The prosthetic heart valve (10, 24, 26, 30) of one of the preceding claim, wherein the number of flaps is two or three.

12. The prosthetic heart valve (10, 24, 26, 30) of one of the preceding claims, wherein the flaps (14a,b,c) are made from biological tissue that is attached to said frame member (12).

13. The prosthetic heart valve (30) of one of the preceding claims, wherein said frame member (12) comprises a shape memory material that can make a reversible transition between two different geometric configurations.
